# EUROPÄISCHE PATENTSCHRIFT

(11) **EP 2 241 275 B1**
(45) Veröffentlichungstag und Bekanntmachung des Hinweises auf die Patenterteilung: **18.09.2013**
(21) Anmeldenummer: 10160035.1
(22) Anmeldetag: 15.04.2010
(51) Int. Cl.: A61B 17/34, A61B 17/02

(54) **Dichtung zum Abschließen eines Zugangsinstrumentes in einen Körper**
Medical instrument for creating an access point for minimally invasive procedures
Instrument médical destiné à créer un accès pour une intervention mini-invasive

(30) Priorität: 17.04.2009 DE 102009018639
(43) Veröffentlichungstag der Anmeldung: 20.10.2010
(73) Patentinhaber: Karl Storz GmbH & Co. KG, 78532 Tuttlingen (DE)
(72) Erfinder: Oberländer, Martin, 78234, Engen (DE); Pilz, Kevin, 78532, Tuttlingen (DE); Sauer, Michael, 78532, Tuttlingen (DE); Wagner, Sebastian, 75015, Bretten (DE)
(74) Vertreter: Witte, Weller & Partner

(56) Entgegenhaltungen:
- WO-A2-2009/035663
- US-A1- 2008 161 826
- US-A1- 2008 255 519
- US-B1- 6 551 270

## Beschreibung

Die Erfindung betrifft eine Dichtung zum Abschließen einer proximalseitigen Zugangsöffnung eines Zugangsinstrumentes in einen Körper, mit einer Kappe, die eine die Zugangsöffnung überdeckende scheibenförmige Wand und eine umfängliche Manschette aufweist, die über den Rand der Zugangsöffnung stülpbar ist, wobei in der Wand zumindest zwei Öffnungen vorhanden sind, wobei auf jeder der Öffnungen ein flexibler Dom sitzt, der proximalseitig eine Eintrittsöffnung zum dichtenden Einführen eines Schaftes eines Instrumentes aufweist.

Eine derartige Dichtung ist aus der US 6,551,270 B1 bekannt.

Weitere Ausgestaltungen von Dichtungen, von denen sich domartige Gebilde erheben, sind aus der US 2008/0161826 A1 und der WO 2009/035663 A2 bekannt.

Aus der US 2008/0161826 A1 ist es bekannt, den Domen eine hohlzylindrische Kegelabschnittform zu verleihen, wobei sich der Kegel in Richtung auf die Wand erweitert. Die Querschnitte der Dome sowie der Öffnungen, von denen diese sich erheben, sind kreisförmig.

In der weit verbreiteten minimalinvasiven Chirurgie ist es bekannt geworden, über ein Zugangsinstrument Zugang zu einem inneren Hohlraum eines Körpers zu erlangen. Derartige Zugangsinstrumente sind Trokare, deren Trokarhülse, bspw. bei der Laparoskopie, durch die Bauchdecke hindurchgestochen wird. Um dies möglichst atraumatisch durchzuführen, wird zunächst in die Haut ein kleiner Einschnitt eingebracht, meist mit einer Länge von 1-1,5 cm, an diesem Einschnitt wird der Trokar mit einem eingeschobenen angespitzten Trokardorn angesetzt und dann durch die Bauchdecke hindurchgeschoben. Nach dem Eingriff wird die Trokarhülse wieder abgezogen und die Inzision verschlossen. Nach einer gewissen Zeit ist, außer einer kleinen Narbe in der Haut, von dem Eingriff äußerlich nichts mehr zu erkennen. Dem Durchmesser der Trokarhülse sind gewisse Grenzen gesetzt, die im Bereich von etwa 15 mm liegen.

Da aber bei einem minimalinvasiven Eingriff bspw. in der Bauchinnenhöhle zahlreiche Instrumente hindurchgeführt werden müssen, ist es üblich geworden zu einem solchen Eingriff mehrere Trokare bspw. an der Bauchdecke anzusetzen, wobei 3 bis 4 Trokare durchaus üblich sind.

In der Weiterführung dieser Zugangstechnologie wurde von der Anmelderin ein Zugangsinstrument in einen Körper entwickelt, das es ermöglicht, insbesondere wenn das Zugangsinstrument durch den Nabel eingesetzt wird, eine wesentlich größere Zugangsöffnung zu schaffen, so dass durch dieses Zugangsinstrument gleichzeitig mehrere Instrumente hindurchgeführt werden können. Das heißt es ist nur ein einziges Zugangsinstrument notwendig, durch dessen einzige relativ große Zugangsöffnung mehrere Instrumente gleichzeitig hindurchgeführt werden können (*Single Port Access*).

Der Körper eines solchen Zugangsinstrumentes ist aus bspw. zwei Teilkörpern zusammengesetzt, wobei jeder Teilkörper einen distalen Teilkörperabschnitt aufweist, der in einen von der Mittellängsachse nach außen abstehenden proximalen Teilkörperabschnitt übergeht. Die distalen Teilkörperabschnitte können in einer ersten Position zu einem distalen Körper mit seitlich abstehenden proximalen Teilkörperabschnitten zusammengesetzt werden. Die beiden zusammengesetzten distalen Teilkörperabschnitte bilden quasi eine Trokarhülse und können, wie zuvor beschrieben, durch die Bauchdecke bei der Laparoskopie hindurchgeschoben werden. Der Durchmesser kann dabei im Bereich von üblichen Trokarhülsen sein. Anschließend werden die beiden seitlich vom Körper abstehenden proximalen Teilkörperabschnitte durch Verschwenken bzw. Aufeinanderzubewegen zu einem proximalen Hohlkörper zusammengesetzt dessen äußerer proximaler Rand die Zugangsöffnung des Zugangsinstrumentes darstellt.

Bei diesem Verschwenken werden die beiden im Körper steckenden und ursprünglich zusammengelegten distalen Teilkörperabschnitte voneinander weg bewegt und verschwenkt. Durch entsprechende Ausgestaltung des Übergangsbereichs zwischen distalem und proximalem Teilkörperabschnitt kann bei diesem Verschwenken die Öffnung in der Bauchdecke noch etwas aufgeweitet werden. Die beiden proximalen Teilkörperabschnitte können an ihrem proximalen Rand eine Öffnung umgrenzen, die einen Durchmesser von mehreren Zentimetern aufweist.

Dadurch ist die Möglichkeit geschaffen durch ein solches einziges Zugangsinstrument gleichzeitig mehrere Instrumente in den Körper einzuschieben.

Nähere Ausgestaltungen eines solchen Zugangsinstrumentes sind in der deutschen Patentanmeldung der Anmelderin vom 13. März 2009, amtliches Aktenzeichen 10 2009 014 525.7, näher beschrieben und erläutert, so dass diesbezüglich auf den Inhalt dieser Patentanmeldung ausdrücklich Bezug genommen wird.

Möchte man ein solches Zugangsinstrument nach proximal abdichten, muss eine Dichtung aufgesetzt werden. Insbesondere bei laparoskopischen Eingriffen wird zum Aufblähen der Bauchdecke und zum Vergrößern des Innenraums durch das Zugangsinstrument ein Insufflationsgas zugeführt. Damit dieses nicht wieder entweicht, ist eine solche Dichtung vorgesehen.

Gegenstand der vorliegenden Erfindung ist somit eine solche Dichtung zum Abschließen einer proximalen Zugangöffnung eines Zugangsinstrumentes in einen Körper.

Aus der EP 1 314 392 B1 der Anmelderin ist eine Dichtung für ein Endoskop bekannt, bei der in eine endseitige auf ein Endoskop aufschraubbare starre Kappe, in deren Wand mehrere Öffnungen vorgesehen sind, unterschiedliche Dichtungen in diese Öffnungen eingesetzt werden. Diese Öffnungen sind alle kreisförmig und die Öffnungen dienen dazu einen Schaft eines Instrumentes, das durch diese Dichtung hindurchgeschoben wird, gasdicht zur Außenseite hin abzudichten. Bei manchen Dichtungen ist die proximalseitige Öffnung, durch die der Schaft eines Instruments dichtend hindurchgeschoben werden soll, kleiner als die Öffnung in der Kappe in die die Dichtung eingesetzt ist. Um eine gewisse seitliche bzw. Kippbewegung des durch die Dichtung eingeschobenen Instrumentes zu ermöglichen, ist dessen Körper als Faltenbalg ausgebildet.

Es ist Aufgabe der vorliegenden Erfindung eine Dichtung zum Abschließen einer proximalseitigen Zugangsöffnung eines Zugangsinstrumentes in einen Körper zu schaffen, die es ermöglicht mehrere Instrumente gleichzeitig durch die Dichtung hindurchzuführen und es zugleich ermöglicht, die durchgeführten Instrumente in einem möglichst großen Bereich beweglich zu handhaben, d.h. hin und her zu bewegen und zu verkippen.

Erfindungsgemäß wird die Aufgabe dadurch gelöst, dass die Öffnungen in der Wand als etwa kreisabschnittförmige Öffnungen ausgebildet sind, zwischen denen ein Materialsteg aus dem Material der Wand vorhanden ist und dass die proximalseitige Eintrittsöffnung jedes Domes kleiner ist als die etwa kreisabschnittförmige Öffnung, über die sich jeweils die Dome erheben.

Diese Maßnahmen haben nun verschiedene Vorteile.

Dadurch, dass die Dichtung eine Kappe aufweist mit einer Wand, die die gesamte Zugangsöffnung des Zugangsinstrumentes überdeckt, ist es möglich, ohne weitere zusätzliche Bauteile, wie Verschlusskappen, Überwurfmuttern oder dergleichen, die gesamte Öffnung abzudichten. Durch die umfängliche Manschette, die über einen Rand der Zugangsöffnung gestülpt ist, ist ein fester und dichtender Sitz der Dichtung an dieser Zugangsöffnung gewährleistet. Die Wand kann dabei Öffnungen überspannen, die Durchmesser im Bereich von mehreren Zentimetern aufweisen, die bis in den Größenbereich von 10 cm gehen.

Durch Vorsehen von zumindest zwei kreisabschnittförmigen Öffnungen, auf denen die flexible Dome sitzen, sind im Bereich der Wand relativ große Öffnungen vorhanden, die einem durch den flexiblen Dom hindurchgeschobenem Instrument einen möglichst großen Aktionsradius ermöglichen. Diese kreisabschnittförmige Öffnung ist dabei um ein Mehrfaches größer als die Querschnittsfläche des schaftartigen Instrumentes, das durch den flexiblen Dom hindurchgeschoben wird. Daher ist proximalseitig am flexiblen Dom eine gegenüber der kreisabschnittförmigen kleinere Öffnung vorhanden, die sich dichtend an den Schaft des Instrumentes anlegt. Der flexible Dom kann als sackartiges Gebilde ausgeformt sein, das am proximalen Ende einen relativ geringen Durchmesser aufweist und sich am distalen Ende zur kreisabschnittförmigen Öffnung aufweitet.

Das heißt, dass das Instrument proximalseitig dichtend durch die relativ kleine Öffnung in den flexiblen Dom eingeschoben werden kann. Aufgrund der wesentlich größeren kreisabschnittförmigen Öffnung in der Wand am Boden des flexiblen Domes, ist aber der gewünschte Aktionsradius ermöglicht. Aktionsradius bedeutet, dass bspw. der Schaft in umfängliche Richtung oder in Richtung einer Sekante längs der kreisabschnittförmigen Öffnung hin- und herbewegt werden kann und auch extreme Kippbewegungen durchführen kann, d.h. der Schaft kann mit einer extremen Neigung durch die Dichtung hindurchgeführt werden. Der flexible Dom kann diesen Bewegungen folgen.

Da mindestens zwei solche kreisabschnittförmige Öffnungen vorhanden sind, ergibt sich für zwei Instrumente ein relativ großer Aktionsbereich, der in umfänglicher Bewegungsrichtung gesehen für jedes Instrument zumindest im Bereich von 90° liegt, zugleich sind noch die zuvor erwähnten Kippbewegungen möglich.

Das heißt durch diesen großen Aktionsbereich kann der Chirurg das distale Ende eines durch diese Dichtung hindurchgeschobenen Instrumentes in einem sehr großen räumlichen Bereich in der Körperhöhle bewegen. Das heißt, er kann trotz eines einzigen Zuganges durch die Bauchdecke (*Single Port Access*) mit mehreren Instrumenten in einem sehr großen Aktionsbereich im Körper hantieren.

Dies stellt einen beachtlichen Fortschritt in der minimalinvasiven Chirurgie dar, der letztendlich dazu führt, dass wesentlich weniger Einschnitte im Körper notwendig sind, um einen minimalinvasiven Eingriff durchzuführen.

In einer weiteren Ausgestaltung der Erfindung ist der flexible Dom als domartiger Faltenbalg ausgebildet.

Die Ausgestaltung als Faltenbalg hat den Vorteil, dass diese Geometrie besonders dazu geeignet ist, die erforderlichen Bewegungen eines in den flexiblen Dom eingeschobenen Instruments zu ermögliche, also seitliche oder Kippbewegungen.

In einer weiteren Ausgestaltung der Erfindung verjüngt sich ein Faltenbalg, quer zu einer sich längs der kreisabschnittförmigen Öffnung erstreckenden Sekante gesehen, tannenbaumartig.

Diese tannenbaumartige Faltenbalgstruktur erlaubt sowohl seitliche Verschiebebewegungen des Faltenbalges und extreme Kippbewegungen ohne dass ein allzu bulkiger Aufbau über der Dichtung notwendig ist. Trotz extremer Verschiebe- und Kippbewegungen ist immer sichergestellt, dass das Instrument gasdicht durch den Faltenbalg hindurchgeführt werden kann, so dass kein Insufflationsgas entweicht demzufolge auch nicht wieder der Körperhöhle zugeführt werden muss.

In einer weiteren Ausgestaltung der Erfindung weist jeder Faltenbalg zumindest drei Falten auf.

Es wurde festgestellt, dass mit drei Falten eine hervorragende Bewegungsfreiheit mit gängigen Materialien, bspw. mit Silikonkautschukmaterialien, erzielt werden kann. Diese Geometrie in Zusammenhang mit dem gewählten Material benötigt keine übermäßige Kräfte um den Faltenbalg zu verformen, außerdem erlaubt die Rückstellkraft des Materials den Faltenbalg in seiner gewünschten Ausrichtung aufzustellen, wenn kein Instrument eingeschoben ist und verschoben oder gekippt wurde.

In einer weiteren Ausgestaltung der Erfindung sind die flexiblen Dome spiegelbildlich zu einem Durchmesser der Wand angeordnet.

Diese Maßnahme hat den Vorteil, dass zwei gegenüberliegende domartige Gebilde zur Verfügung stehen, die dann einen maximal großen Aktionsradius erlauben, dennoch aber zwischen den Domen genügend Material stehenbleiben kann, um für die ausreichende mechanische Festigkeit der Dichtung zu sorgen. Es ist zu beachten, dass entgegen dem eingangs erwähnten Stand der Technik die Dichtung nicht nur eine relativ kleine Durchgangsöffnung abdichtet, sondern eine relativ große im Durchmesser von mehreren Zentimetern erstreckende Zugangsöffnung des Zugangsinstrumentes.

In einer weiteren Ausgestaltung der Erfindung ist zwischen den flexiblen Domen zumindest eine weitere Öffnung in der Wand ausgebildet.

Diese Maßnahme hat nun den erheblichen Vorteil, dass in diesem Bereich weitere Instrumente hindurchgeführt werden können, die nicht diesen Aktionsradius benötigen, also keinen Dom notwendig haben, sondern lediglich eine Durchtrittsöffnung benötigen. Solche Öffnungen sind bspw. Öffnungen für Gasanschlüsse oder dergleichen.

Wie zuvor erwähnt, erlaubt die diametrale spiegelbildliche Anordnung der beiden Dome zwischen diesen noch einen relativ großen Bereich an Wandmaterial, so dass in diesem Bereich zwischen den Domen auch noch weitere Öffnungen vorgesehen werden können, ohne die mechanische Stabilität der Dichtung zu beeinträchtigen.

In einer weiteren Ausgestaltung ist in einer ersten weiteren Öffnung ein Zugang für ein weiteres Instrument, insbesondere eine Optik, geschaffen.

Diese Maßnahme hat den Vorteil, dass bspw. eine Optik eingebracht werden kann, die den gesamten Innenhohlraum erfasst, so dass dann über diese stillstehende Optik, die Manipulationen, mit den durch die Dome durchgeführten bewegbaren Instrumente beobachtet werden kann.

Bei der bislang üblichen minimalinvasiven Chirurgie hätten dazu drei Trokare gesetzt werden müssen, einer für die Optik und zwei für die Instrumente, wobei die Trokare für die Instrumente eben nicht einmal diese Aktionsbereiche erlauben.

Hierin ist ein besonderer Vorteil der erfindungsgemäßen Dichtung zu sehen.

In einer weiteren Ausgestaltung der Erfindung ist in einer zweiten weiteren Öffnung ein Gasanschluss eingesetzt.

Diese Maßnahme hat den Vorteil, dass in diesem Bereich auch noch zusätzlich das Insufflationsgas durch die Dichtung hindurchgeführt werden kann.

Bei Trokaren ist üblicherweise ein Trokargehäuse vorhanden, von dem seitlich ein Anschluss vorsteht, an den das Insufflationsgas angeschlossen werden kann. Die zuvor erwähnte vorteilhafte Ausgestaltung der Dichtung erlaubt somit die notwendigen Instrumente, die Optik und den Gasanschluss durch die Dichtung hindurchzuführen, so dass das eigentliche Zugangsinstrument keine solchen Anschlüsse mehr aufweisen muss, entsprechend einfach aufgebaut werden kann, ggf. als einmalig zu benutzendes Wegwerfteil.

In einer weiteren Ausgestaltung der Erfindung ist in eine weitere Öffnung eine Reduzierhülse eingesetzt, die proximalseitig eine durchmesserkleinere Öffnung als diese Öffnung aufweist.

Diese Maßnahme hat den Vorteil, dass man eine bestimmte Grundgröße einer weiteren Öffnung vorsehen kann. Falls wesentlich dünnere Schäfte durchgeführt werden sollen, wird eine Reduzierhülse eingesetzt und durch diese wird dann das zusätzliche Instrument bspw. eine Optik hindurchgeführt.

In einer weiteren Ausgestaltung der Erfindung ist in der Kappe eine Klammer integriert.

Diese Maßnahme hat den Vorteil, dass mittels der Klammer die Dichtung besonders fest auf dem Rand der Zugangsöffnung befestigt werden kann.

In einer weiteren Ausgestaltung der Erfindung ist die Klammer als diametraler Bügel ausgebildet, dessen endseitige Krallen am Rand des Zugangsinstrumentes anliegen.

Dabei ist von Vorteil, den diametralen Bügel direkt in das Material der Dichtung miteinzubetten, bspw. bei Ausbildung als Silikondichtung direkt beim Ausformen der Dichtung miteinzugießen. In diesem Bereich, der zwischen den beiden spiegelbildlich gegenüberliegenden flexiblen Domen liegen kann, wird dann durch den Bügel eine zusätzliche Verstärkung gebildet, so dass dieser Steg zwischen den Domen extrem schmal, ausgebildet werden kann, denn dieser Bügel trägt zu Stabilität bei. Dadurch ist es dann möglich, den Aktionsbereich am Boden der Dome möglichst großflächig auszugestalten.

In einer weiteren Ausgestaltung der Erfindung ist in einem proximalseitigen Kragen eines flexiblen Domes eine Schlitzdichtung angeordnet.

Diese Maßnahme hat den Vorteil, dass die proximalseitigen Enden der Dome durch die Schlitzdichtungen abgedichtet sind, wenn kein Instrument durchgeschoben ist, oder wenn bspw. nur durch einen der zumindest zwei Dome ein Instrument hindurchgeschoben ist.

In einer weiteren Ausgestaltung der Erfindung sind distalseitig an den weiteren Öffnungen Schlitzdichtungen angeordnet.

Diese Maßnahme hat denselben Effekt, wie zuvor erwähnt, d.h. diese Schlitzdichtungen dichten die weiteren Öffnungen ab, wenn diese entweder nicht benötigt werden, also kein Instrument durchgeschoben wird, oder während ein Instrument abgezogen und durch ein anderes ersetzt wird.

Insgesamt gesehen, sind somit sämtliche Zugangsöffnungen bzw. Durchtrittsöffnungen entsprechend abgedichtet, so dass, selbst wenn kein Instrument eingesteckt ist, ein gasdichter Abschluss vorhanden ist.

Dieser öffnet bspw. die Möglichkeit, zur Vorbereitung eines minimalinvasiven Eingriffes, die Dichtung zunächst auf die Zugangsöffnung des Zugangsinstrumentes aufzusetzen, indem die Manschette übergestülpt wird und über einen Gasanschluss ein Insufflationsgas durch die Dichtung in den Körper einzuführen. Dann kann man nach und nach durch die flexiblen Dome die Instrumente hindurchführen und den eigentlichen Eingriff beginnen.

In einer weiteren Ausgestaltung der Erfindung ist die Klammer derart ausgebildet, dass Teilkörperabschnitte eines die Zugangsöffnung des Zugangsinstrumentes umgebenden Randes zusammenhaltbar sind.

Wie eingangs erwähnt, ist ein vorteilhafter Anwendungsbereich der Dichtung ein Zugangsinstrument, das aus zwei umklappbaren Teilkörperabschnitten zusammengesetzt ist, geeignet. Die Manschette der Dichtung, die über den Rand gestülpt wird, hat schon eine gewisse Haltefunktion, d.h. diese hält diese beiden proximalen vom Körper abstehenden Teilkörperabschnitte zusammen.

Bei extrem großen Öffnungen oder starker Aufweitung der Körperöffnungen und bei extrem steifen oder festen Bauchdecken, wirken erhebliche Kräfte in Richtung Trennen bzw. Spreizen der zusammengelegten proximalen Teilkörperabschnitte.

Durch Vorsehen einer Klammer in der Kappe können nun diese Teilkörperabschnitte zusätzlich durch diese Klammer aneinandergehalten werden. Dies stellt eine besonders vorteilhafte Ausgestaltung bei dem Anwendungsgebiet der aus Teilkörperabschnitten zusammengesetzten Zugangsinstrumente dar.

In einer weiteren Ausgestaltung der Erfindung steht von der Kappe zumindest ein Vorsprung vor, der ergreifbar ist und ein Aufstülpen der Kappe auf den Rand der Zugangsöffnung erleichtert.

Diese Maßnahme hat den Vorteil, dass die Kappe an dem Vorsprung ergriffen werden kann, sei es von Hand oder mit einem Werkzeug und durch Ziehen an dem Vorsprung etwas aufgeweitet werden kann, wodurch das Aufstülpen oder Aufsetzen der Kappe auf den Rand erleichtert wird.

In einer weiteren Ausgestaltung der Erfindung sind mehrere Vorsprünge umfänglich verteilt vorgesehen.

Diese Maßnahme hat den Vorteil, dass die Kappe an unterschiedlichen oder gleich an mehreren Stellen ergriffen und entsprechend aufgeweitet werden kann, was den Aufstülpvorgang wesentlich erleichtert.

In einer weiteren Ausgestaltung der Erfindung springt der zumindest eine Vorsprung radial nach außen gerichtet von einem unteren Ende der Manschette vor.

Diese Maßnahme hat den Vorteil, dass das Überstülpen der umfänglichen Manschette besonders erleichtert wird, das heißt, das nur diese aufgeweitet oder gedehnt wird, ohne dabei die weitere Dichtung, insbesondere die die Zugangsöffnung überdeckende Wand, beeinträchtigt werden muss.

In einer weiteren Ausgestaltung der Erfindung ist der zumindest eine Vorsprung als Lasche ausgebildet.

Diese Maßnahme hat den Vorteil, dass eine solche Lasche bspw. einfach zwischen zwei Fingern ergriffen und an dieser gezogen werden kann, so dass die Manschette so weit gedehnt werden kann, dass sie einfach über den Rand der Zugangsöffnung gestülpt werden kann. Bei der Ausgestaltung mit mehreren Laschen kann an zwei oder mehreren diametral gegenüberliegenden Laschen gezogen werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird anhand einiger ausgewählter Ausführungsbeispiele in Zusammenhang mit den beiliegenden Zeichnungen näher beschrieben und erläutert. Es zeigen:
- Fig. 1: perspektivisch ein erstes Ausführungsbeispiel einer erfindungs- gemäßen Dichtung;
- Fig. 2: einen Schnitt längs der Linie II-II in Fig. 1;
- Fig. 3: eine der Fig. 2 vergleichbare Darstellung, wobei ein Schaft eines Instrumentes in einen domartigen Faltenbalg eingeschoben und seitlich aus einer normalen Ausrichtung verschoben ist;
- Fig. 3a: eine der Fig. 3 vergleichbare Darstellung, bei der der flexible Dom als glattwandiger flexibler sackartiger Körper ausgebildet ist;
- Fig. 4: eine der Fig. 3 vergleichbare Darstellung bei der ein Kippen des im domartigen Faltenbalg aufgenommenen Schaftes ersichtlich ist;
- Fig. 5: einen Schnitt längs der Linie V-V in Fig. 1;
- Fig. 6: einen Schnitt längs der Linie VI-VI in Fig. 1;
- Fig. 7: eine perspektivische Ansicht eines Zusammenbaus aus der erfindungsgemäßen Dichtung von Fig. 1 mit einem Zugangsin- strument, wobei in die Dichtung zusätzlich ein Gasanschluss eingesetzt und außerdem eine Reduzierhülse sind;
- Fig. 8: einen Vertikalschnitt durch den Zusammenbau von Fig. 7, wobei dargestellt ist wie dieser Zusammenbau in eine Bauchde- cke eines menschlichen Körpers eingesetzt ist;
- Fig. 9: perspektivisch, teilweise aufgeschnitten, eine Ansicht des dista- len Endes eines Ausführungsbeispiels einer erfindungsgemäßen Dichtung mit einer integrierten Klammer,
- Fig. 10: eine perspektivische Darstellung bei der die Dichtung von Fig. 9 auf ein Zugangsinstrument, das aus zwei Teilhälften zusammen- gesetzt, aufgesetzt ist und dabei durch die Klammer diese beiden Teilhälften zusammenhält,
- Fig. 11: eine perspektivische Explosionsdarstellung eines zweiten Aus- führungsbeispiels einer Dichtung,
- Fig. 12: die Dichtung von Fig. 11 in zusammengebautem Zustand und auf einem Zugangsinstrument montiert, und
- Fig. 13: eine Draufsicht auf die Dichtung von Fig. 12.

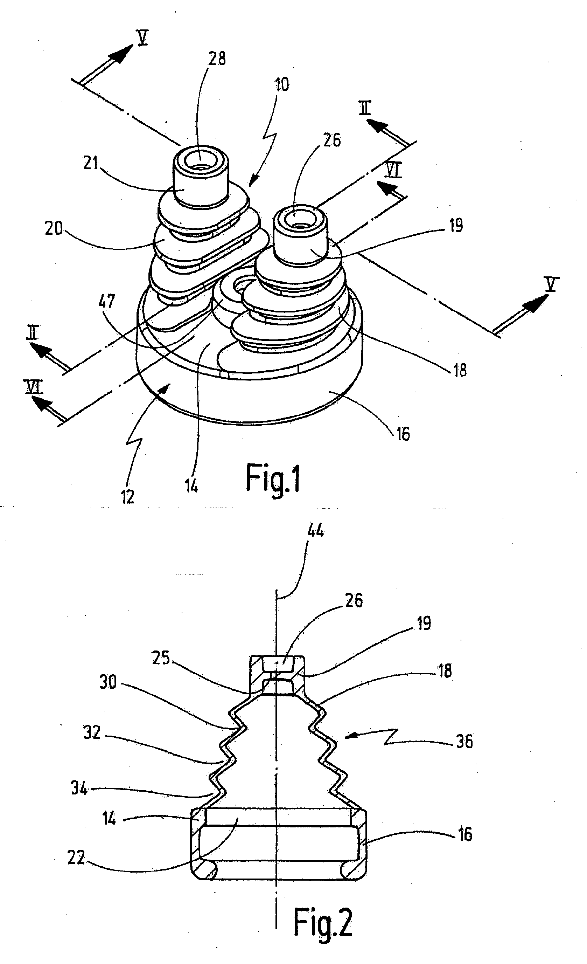

Ein in den Figuren 1-6 dargestelltes erstes Ausführungsbeispiel einer Dichtung ist in ihrer Gesamtheit mit der Bezugsziffer 10 bezeichnet.

Die Dichtung 10 besteht aus einem elastischen Silikonkautschukmaterial und ist durch einen Spritzgussformgang hergestellt.

Die Dichtung 10 weist einen ersten Abschnitt in Form einer Kappe 12 auf, die eine scheibenförmige, etwa ebene Wand 14 aufweist, von der nach proximal umfänglich eine Manschette 16 vorsteht. Von der Wand 14 erheben sich nach proximal zwei domartiger Faltenbälge 18 und 20. Aus der Schnittdarstellung in Fig. 2 ist ersichtlich, dass der domartige Faltenbalg 18 auf dieser Seite, von distal nach proximal gesehen, eine tannenbaumartige Verjüngung 36 aufweist und drei Falten 30, 32 und 34 aufweist. Am proximalen Ende ist ein zylindrischer Kragen 19 vorhanden, der eine Eintrittsöffnung 26 umrundet. Im Kragen 19 ist eine Schlitzdichtung 25 angeordnet.

Im Nachfolgenden sind faltenbalgartige Dome beschrieben, es können auch sackartige flexible Dome sein, die dieselben Bewegungen erlauben, wie das in Zusammenhang mit den domartigen Faltenbälgen 18 und 20 beschrieben wird.

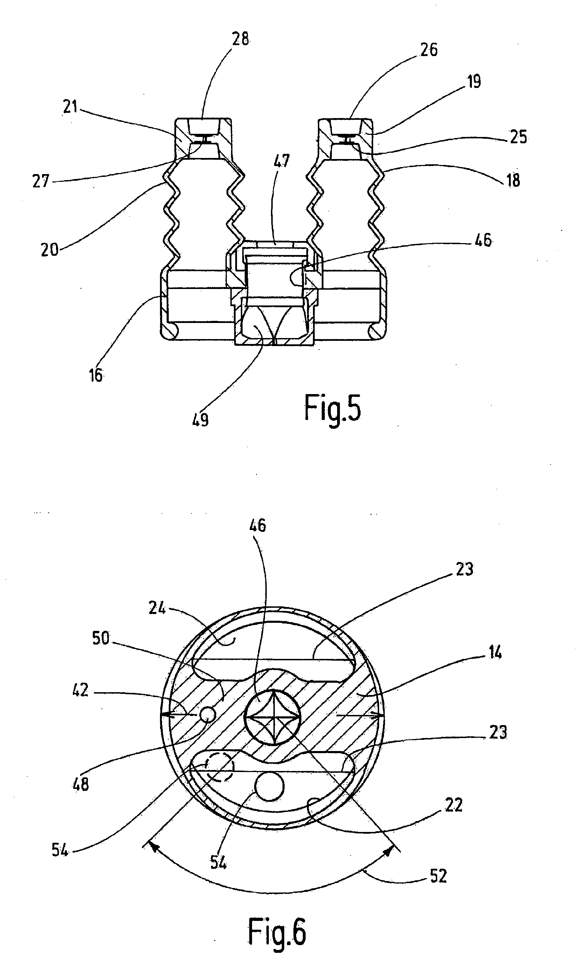

Aus der perspektivischen Darstellung von Fig. 1 ist ersichtlich, dass auch der dem domartigen Faltenbalg 18 gegenüberliegende domartige Faltenbalg 20 gleichermaßen ausgebildet ist, nur eben spiegelbildlich dazu, wobei sich die Spiegelebene längs eines Durchmessers 42 erstreckt, wie er aus Fig. 6 ersichtlich ist. In Fig. 6 ist eine Draufsicht von proximal nach distal auf die Wand 14 ersichtlich. Aus dieser Schnittdarstellung ist ersichtlich, dass in der Wand 14 zwei grob kreisabschnittförmige Öffnungen 22, 24 ausgespart sind. Diese beiden Öffnungen 22, 24 bilden quasi den offenen Boden der domartigen Faltenbälge 18 und 20.

Die beiden Faltenbälge 18 und 20 erheben sich über den Öffnungen 22 und 24.

Aus der Schnittdarstellung von Fig. 5 ist ersichtlich, dass die beiden Faltenbälge 18 und 20 in einem radialen Abstand zueinander angeordnet sind. Aus der Darstellung von Fig. 6 ist zu erkennen, dass dazwischen ein Materialsteg 50 vorhanden ist, der für eine ausreichende mechanische Stabilität der Wand 14 sorgt. Aus Fig. 6 ist zu erkennen, dass im Steg 50 eine erste weitere Öffnung 46 vorhanden ist, die, wie das aus Fig. 5 ersichtlich ist, distalseitig mit einer Kreuzschlitzdichtung 49 abgeschlossen ist.

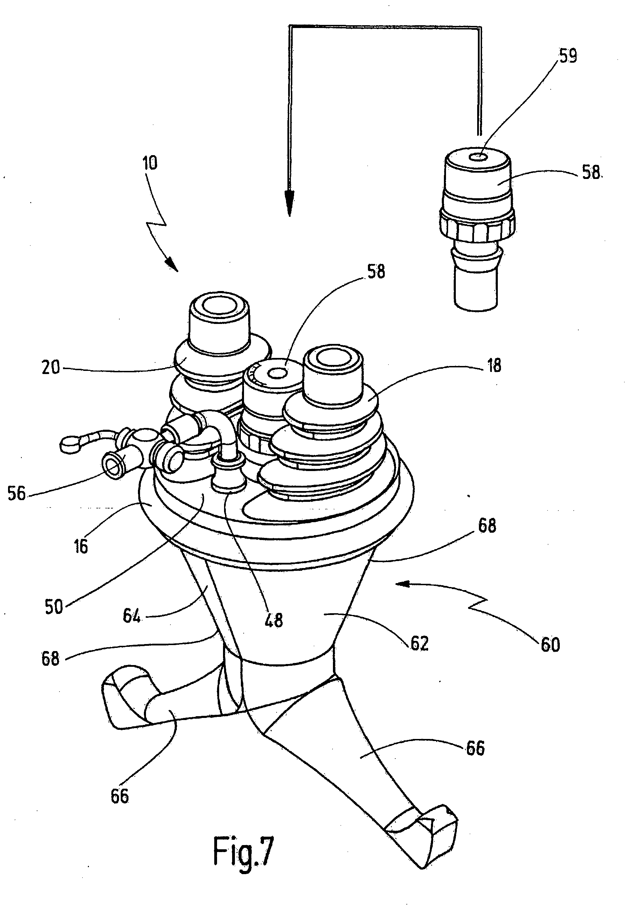

Eine zweite weitere Öffnung 48, die wesentlich kleiner ist, dient dazu, in diese einen Gasanschluss 56 einzustücken, wie er aus Fig. 7 ersichtlich ist.

In der ersten weiteren Öffnung 46 ist proximalseitig eine zylindrische Steckdichtung 47 eingesteckt, die einen bestimmten maximalen Einführöffnungsdurchmesser bestimmt.

In Zusammenhang mit den Figuren 1-6 soll ein besonderer Vorteil bei der Handhabung der Dichtung 10 beschrieben werden.

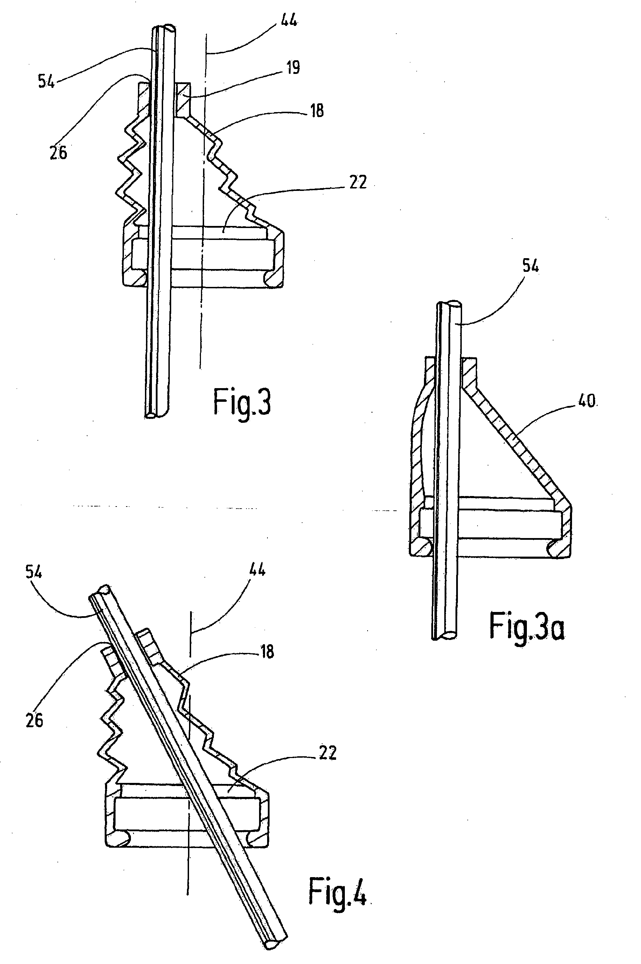

In Fig. 3 ist dargestellt, wie durch den domartigen Faltenbalg 18 ein Schaft 54 eines Operationsinstrumentes hindurchgeschoben ist. Normalerweise steht der Schaft 54 in Ausrichtung mit der Mittellängsachse 44 des Domes, wie das in Fig. 2 dargestellt ist. Aufgrund der Faltenbalgkonstruktion kann nun bspw. der Schaft 54 aus der Ausrichtung mit der Mittellängsachse 44 von dieser nach rechts oder nach links bewegt werden, wie das in Fig. 3 dargestellt ist.

Dies ist zum einen durch die Faltenbalgkonstruktion möglich und zum anderen, dass die in Fig. 6 ersichtliche etwa kreisabschnitt- oder nierenförmige Öffnung 22 vorhanden ist. Es ist einleuchtend, dass diese Bewegungen auch durch sackartige Dome, ohne Falten, ermöglicht sind. Die Säcke müssen so ausgebildet sein, dass die in Fig. 3 und Fig. 4 dargestellten Positionen ebenfalls möglich sind.

Die in Fig. 6 mit einem durchgezogenen Kreis bezeichnende Position des Schafts 54 entspräche der Ausrichtung, wenn der Schaft in den Faltenbalg 18, wie er in Fig. 2 dargestellt ist, eingesteckt wäre.

Die in Fig. 6 mit gestrichelten Linien angedeutete Position des Schaftes 54 entspricht der in Fig. 3 dargestellten Position d.h. einer maximalen Verschiebeposition nach links. Die Länge des Verschiebewegs entspricht der Länge einer Sekante 23 der kreisabschnittförmigen Öffnung 22.

Aus Fig. 3a ist eine weitere Ausführungsform ersichtlich, dort ist ein flexibler Dom 40 aus einem sackartigen, glattwandigen elastischen Kunststoff ausgebildet.

Das Material ist so flexibel, dass dieselben Bewegungen möglich sind, wie dies in Zusammenhang mit dem domartigen Faltenbalg beschrieben ist.

Aus Fig. 4 ist zu erkennen, dass der Schaft 54 nicht nur hin- und herverschoben werden kann, sondern auch relativ extrem gekippt werden kann. Aus Fig. 4 in Zusammenhang mit Fig. 6 ist ersichtlich, dass in der Position der Schaft 54 so weit verkippt werden kann, bis er an das in der Darstellung von Fig. 6, rechte Ende der Öffnung 22, stößt.

Die Kombination der Bewegungen wie sie in Fig. 3 und Fig. 4 dargestellt sind, ergeben somit einen erheblichen Aktionsbereich 52, wie er in Fig. 6 dargestellt ist. Es ist einleuchtend, dass ein distales Ende des Schafts aufgrund dieser Ausgestaltung einen sehr großen Raum im Körper eines Patienten erreichen kann.

Dies gilt natürlich auch für einen weiteren Schaft, der in den anderen Faltenbalg 20 eingeschoben ist, sowie für die Ausgestaltung als Dom 40 wie in Fig. 3a dargestellt.

In Fig. 7 ist eine Situation dargestellt, bei der die Dichtung 10 auf ein Zugangsinstrument 60 aufgesetzt ist.

Das Zugangsinstrument 60 ist aus zwei Teilkörperabschnitten 62 und 64 zusammengesetzt.

Jeder dieser Teilkörperabschnitte 62 und 64 weist einen distalen Körperabschnitt 66 und einen gegenüber diesem seitlich abstehenden proximalen Körperabschnitt 68 auf. In Fig. 7 ist eine Situation dargestellt, bei der die beiden proximalen Teilkörperabschnitte 68 zu einem sich nach proximal relativ stark erweiternden konischen Hohlkörper zusammengesetzt sind.

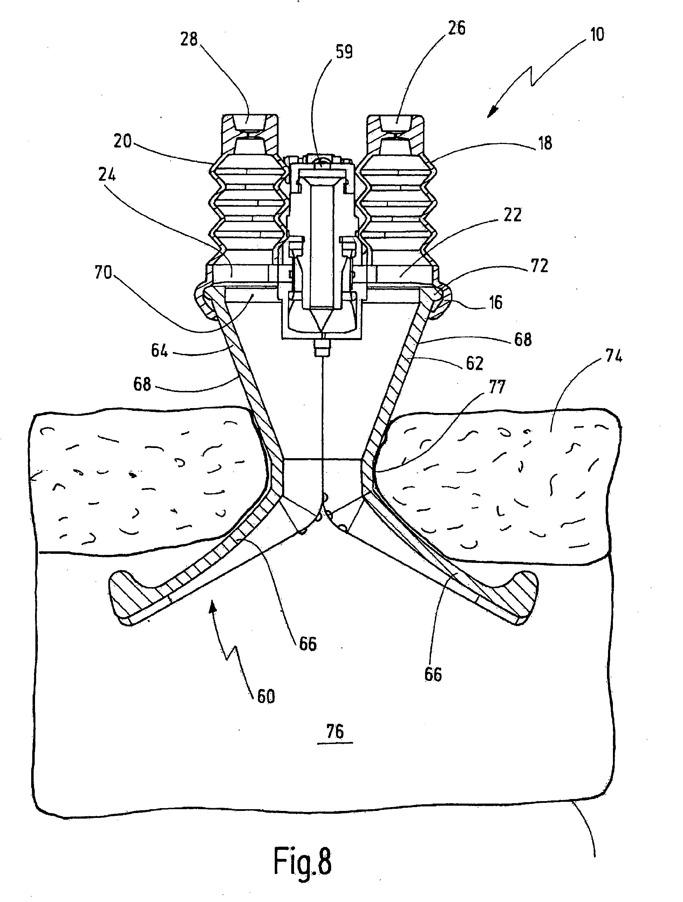

Aus der Schnittdarstellung von Fig. 8 ist ersichtlich, dass dieser proximale Hohlkörper eine relativ große Zugangsöffnung 70 umrundet, auf deren Rand 72 die Dichtung 10 aufgesetzt ist. Dabei ist die Manschette 16 der Dichtung 10 über den Rand 72 der Zugangsöffnung 70 des Zugangsinstrumentes 60 gestülpt. Die Ausformung und die Wahl des elastischen Materials sind so, dass nach Überstülpen der Manschette 16 über den Rand 72 dieser gasdicht durch die Dichtung 10 abgeschlossen ist. In Fig. 7 ist ersichtlich, dass in die zweite weitere Öffnung 48 ein Gasanschluss 56 eingesetzt ist, und außerdem ist in die in Fig. 1 ersichtliche zylindrische Steckdichtung 47 noch eine Reduzierhülse 58 eingesteckt. Diese Reduzierhülse 58 weist eine kleinere Öffnung 59 auf, so dass durch diese ein entsprechend dünnschaftiges Instrument hindurchgeführt werden kann.

Aus der perspektivischen Darstellung von Fig. 7 in Zusammenhang mit Fig. 8 ist ersichtlich, dass über die Dichtung 10 bzw. durch deren Wand 14 über den Gasanschluss 56 ein Insufflationsgas zugeführt werden kann, über die domartige Faltenbälge 18 und 20 Schäfte 54 von entsprechenden Operationsinstrumenten hindurchgeführt werden können und durch die Reduzierhülse 58 ein weiteres Instrument, bspw. eine Optik, durchgeführt werden kann. All die durch die Dichtung 10 hindurchgeführten Instrumente werden durch den proximalen Hohlkörper, der aus den beiden Teilkörperabschnitten 68 zusammengesetzt ist, geführt und reichen, nachdem das Zugangsinstrument 60 in einen Körper 75 eingesetzt ist, in dessen Innenraum, bspw. in den Bauchraum 76, hinein.

Das Zugangsinstrument 60 wird so in den Körper 75 bzw. durch die Bauchdecke 74 hindurchgeführt, dass es zunächst ohne die Dichtung 10 versehen ist und die beiden distalen Teilkörperabschnitte 66 zu einem relativ schmalen Körper zusammengesetzt sind, wie das in der eingangs erwähnten deutschen Patentanmeldung der Anmelderin beschrieben ist. In diesem Bauzustand werden die beiden aneinanderliegenden distalen Teilkörperabschnitte 66 durch die Bauchdecke 74 hindurchgetrieben. Anschließend werden die beiden Teilkörperabschnitte 62 und 64 gegeneinander verschwenkt oder so verkippt, dass die beiden proximalen Teilkörperabschnitte 68 aneinander zum Liegen kommen, und die bereits im Innern des Körpers liegenden distalen Teilkörperabschnitte 66 so gespreizt sind, wie das in Fig. 8 dargestellt ist, also beinahe an der Unterseite der Bauchdecke 74 anliegen. In diesem Bauzustand kann nun die Dichtung 10 aufgesetzt werden, indem deren Manschette 16 über den Rand 72 gestülpt wird.

Beim Übergang von der zuvor erwähnten Position der aneinanderliegenden distalen Teilkörperabschnitte 66 zu der in Fig. 8 gezeigten gespreizten Position wird auch die Öffnung 77 in der Bauchdecke 74 gespreizt, so dass erhebliche Rückstellkräfte auf die beiden gespreizten Teilkörperabschnitte 66 einwirken können, die wieder ein Spreizen der proximalen Teilkörperabschnitte 62 verursachen könnte. Dieser Spreizbewegung wirkt die übergestülpte Manschette 16 der Dichtung 10 entgegen. Da aber auch für einen gasdichten Abschluss längs der Trennlinie der beiden Teilkörperabschnitte 62 und 64, insbesondere im Bereich der außerhalb des Körpers liegt, gesorgt werden soll, ist es notwendig, diese beiden Teile fest aneinander zu halten.

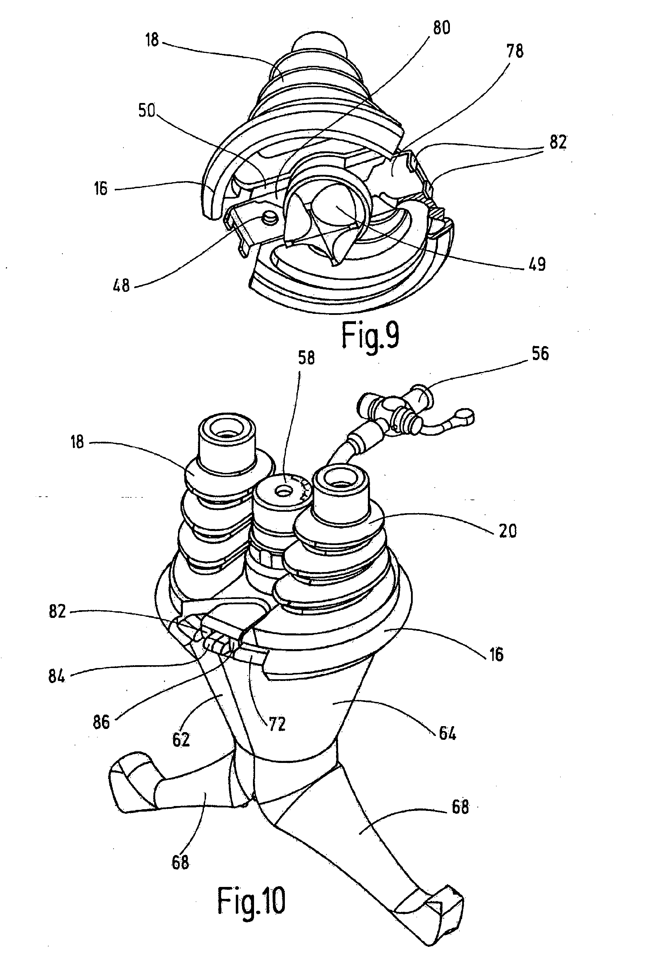

Dazu ist in dem in Fig. 9 und 10 dargestellten Ausführungsbeispiel einer erfindungsgemäßen Dichtung in der Wand 14 eine Klammer 78 integriert. Die Klammer 78 weist einen Bügel 80 auf, der sich diametral erstreckt, und zwar im Bereich des Steges 50 zwischen den beiden domartigen Faltenbälgen 18 und 20. Aus der Darstellung von Fig. 9 ist ersichtlich, dass in dem Steg auch die zweite weitere Öffnung 48 für den Gasanschluss ausgebildet ist und auch die hier nicht ersichtliche erste weitere Öffnung für die zylindrische Steckdichtung 47, da diese durch die distalseitige Kreuzschlitzdichtung 49 abgedeckt ist. Der Bügel, der bspw. aus Metall hergestellt werden kann und bei der Herstellung der Dichtung gleich miteingearbeitet bzw. eingespritzt werden kann, sorgt für eine weitere Versteifung der Dichtung im Bereich der Wand 14.

Endseitig weist der Bügel 80 Krallen 82 auf, die, wie das insbesondere in Fig. 10 ersichtlich ist, in Nuten 86 beidseits von Höckern 84 am Rand 72 eingreifen. Die übergreifenden Krallen 82 halten dann die beiden Teilkörperabschnitte 62 und 64 fest und somit dann auch gasdicht aneinander.

Somit wird von der Dichtung nicht nur die eigentliche Dichtaufgabe bewerkstelligt, sondern zusätzlich noch eine Haltefunktion als Halterung für die beiden Teilkörperabschnitte 62 und 64 des Zugangsinstrumentes 60.

Nach Durchführen eines minimalinvasiven Eingriffes durch die Dichtung 10 hindurch, kann diese wieder von dem Zugangsinstrument abgenommen werden, indem die übergestülpte Manschette 16 abgezogen wird. Nach Entfernen der Dichtung 10 kann dann das Zugangsinstrument wieder umgeklappt werden, so dass die beiden im Körper steigenden distalen Teilkörperabschnitte 66 aneinanderliegen und dann durch die Bauchdecke 74 abgezogen werden können.

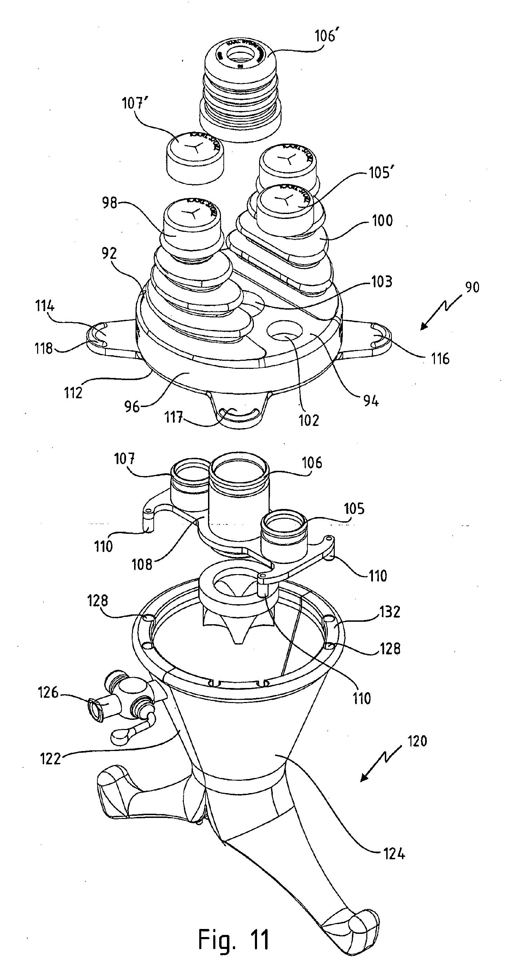

Ein in den Figuren 11 bis 13 dargestelltes weiteres Ausführungsbeispiel einer erfindungsgemäßen Dichtung ist in seiner Gesamtheit mit der Bezugsziffer 90 bezeichnet.

Die Dichtung 90 ist aus einem ähnlichen dehnbaren Material wie die zuvor beschriebene Dichtung 10 hergestellt.

Die Dichtung 90 weist ebenfalls eine Kappe 92 auf, die eine scheibenförmige ebene Wand 94 aufweist, von deren äußerem Rand, in der Darstellung von Fig. 11 nach unten, eine umfängliche Manschette 96 vorspringt.

Von der Wand 94 stehen zwei domartige Faltenbalge 98 und 100 hoch, wie sie zuvor im Zusammenhang mit der Dichtung 10 beschrieben worden sind.

In dem Bereich zwischen den beiden hochstehenden Faltenbalgen 98 und 100 sind drei weitere Öffnung vorgesehen.

In diesen drei weiteren Öffnungen 102, 103, 104 sind drei Dichtungen 105, 106, 107 eingesetzt. Es ist insbesondere aus Fig. 11 zu erkennen, dass drei rohrabschnittförmige Körper der Dichtungen 105, 106, 107 auf einer Klammer 108 montiert sind, von deren Unterseite Zapfen 110 vorspringen.

Diese Zapfen 110 können in Ausnehmungen 128 eingeschoben werden, die am oberen Rand 132 eines aus zwei Teilkörperabschnitten 122 und 124 zusammengesetzten Zugangsinstrument 120 ausgenommen sind.

Dadurch erfüllt die Klammer 108 auch die zuvor erwähnte Aufgabe der Klammer 78, nämlich die beiden Teilkörperabschnitte 122 und 124 in diesem Bauzustand aneinander zu halten.

Bei der Dichtung 90 springen von einem unteren Ende 112 der Manschette 96 radial nach außen gerichtet vier umfänglich gleichmäßig verteilte Laschen 114, 115, 116 und 117 vor.

Diese Laschen 114 bis 117 stellen Vorsprünge dar, anhand derer die Dichtung 90 ergriffen und zum Montage etwas gedehnt werden kann, indem nämlich an den Laschen gezogen wird. Dadurch wird ein Überstülpen der Manschette 96 über den Rand 132 des Zugangsinstruments 120 erleichtert.

Die äußeren Enden der Laschen 114 bis 117 sind mit Wülsten 118 versehen, die ein Abgleiten eines Fingers, der eine solche Lasche ergriffen hat, hindern. Es können auch an der Unterseite entsprechende Wülste 118 vorgesehen sein. Bei der Montage wird die Klammer 108 auf den oberen Rand 132 gesetzt und anschließend die Kappe 92 so aufgesetzt, dass die drei hochstehenden Dichtungen 105, 106, 107 durch die entsprechenden Öffnungen 103, 104, 105 durchgeschoben werden.

Zum Erleichtern des Überstülpens der Manschette 96 kann an den Laschen 114 bis 117 radial nach außen gerichtet gezogen werden.

Es kann auch die Montage so erfolgen, dass die Kappe 92 und die Klammer 108 zunächst zusammengesetzt werden und dann dieser Zusammenbau auf den Rand 132 aufgesetzt wird, wie das der Handhabungsperson am günstigsten ist.

Auf die durch die drei Öffnungen 102, 103, 104 dann durchreichenden Dichtungen 105, 106, 107 werden entsprechende Dichtungskappen 105', 106' und 107' aufgesetzt.

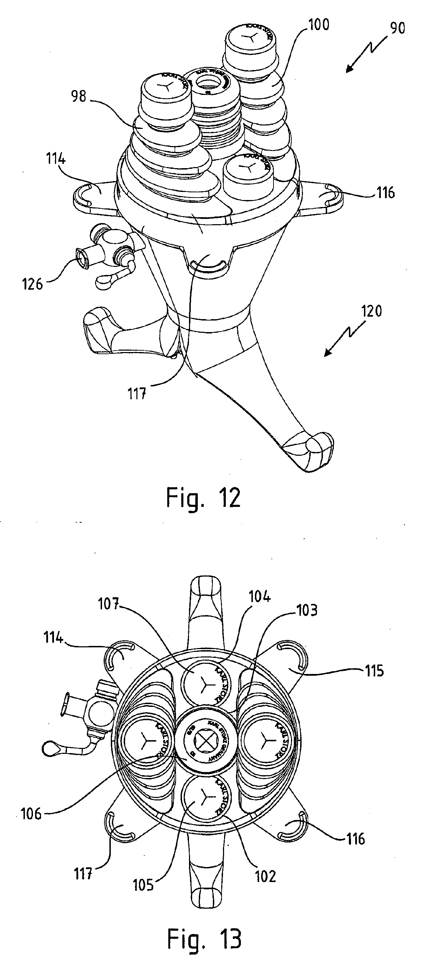

In den Figuren 12 und 13 ist der endmontierte Zustand gezeigt.

Es ist zu erkennen, dass bei dieser Ausführungsform vom Teilkörperabschnitt 122 seitlich ein Luer-Anschluss 126 vorsteht, über den Medien, sei es gasförmige oder flüssige, durch das Zugangsinstrument 120 zugeführt werden können.

Nach einem Einsatz kann die Kappe 92 sehr einfach dadurch vom Zugangsinstrument 120 abgenommen werden, dass an einer oder mehreren der Laschen 114 bis 117 gezogen wird und die Kappe 92 nach oben wegbewegt wird.

Der Einsatz der Dichtung 10 bzw. 90 wurde hier in Zusammenhang mit einem speziell ausgebildeten Zugangsinstrument 60 bzw. 120 beschrieben, es ist aber einleuchtend, dass die Dichtung auf ein anderes Zugangsinstrument aufgesetzt werden kann, das einen entsprechenden Durchmesser aufweist. Wichtig ist, dass durch diese einzige Dichtung eine großflächige Abdichtung dieser relativ großen Zugangsöffnung bewerkstelligt werden kann und durch diese Dichtung zahlreiche Manipulationen durchgeführt werden können, nämlich die operativen Vorgänge durch die Faltenbalge hindurch, der Insufflationsgasfluss durch den Gasanschluss und eine visuelle Beobachtung durch eine mittig in die Dichtung eingesteckte Optik.

Das Prinzip des *Single Port Access* ist nun dahingehend weiterentwickelt worden, dass durch eine einzige diesen *Single Port Access* abschließende Dichtung die gesamten Manipulationen durchgeführt werden können.

## Patentansprüche

1. Dichtung zum Abschließen einer proximalseitigen Zugangsöffnung eines Zugangsinstrumentes (60; 120) in einen Körper (75), mit einer Kappe (12; 92), die eine die Zugangsöffnung (70) überdeckende scheibenförmige Wand (14; 94) und eine umfängliche Manschette (16; 96) aufweist, die über den Rand der Zugangsöffnung stülpbar ist, wobei in der Wand zumindest zwei Öffnungen vorhanden sind, wobei auf jeder der Öffnungen ein flexibler Dom sitzt, der proximalseitig eine Eintrittsöffnung zum dichtenden Einführen eines Schaftes eines Instrumentes aufweist, **dadurch gekennzeichnet, dass** die Öffnungen (22, 24) in der Wand (14; 94) als etwa kreisabschnittförmige Öffnungen (22, 24) ausgebildet sind, zwischen denen ein Materialsteg (50) aus dem Material der Wand (14) vorhanden ist und dass die proximalseitige Eintrittsöffnung (26, 28) jedes Domes (40) kleiner ist als die etwa kreisabschnittförmige Öffnung, (22, 24) über die sich jeweils die Dome (40) erheben.

2. Dichtung nach Anspruch 1, **dadurch gekennzeichnet, dass** der flexible Dom als domartiger Faltenbalg (18, 20; 98, 100) ausgebildet ist.

3. Dichtung nach Anspruch 2, **dadurch gekennzeichnet, dass** sich der Faltenbalg (18, 20; 98, 100), quer zu einer sich längs der kreisabschnittförmigen Öffnungen (22, 24) erstreckenden Sekante (23) gesehen, tannenbaumartig (36) verjüngt.

4. Dichtung nach Anspruch 2 oder 3, **dadurch gekennzeichnet, dass** jeder Faltenbalg (18, 20; 98, 100) zumindest drei Falten (30, 32, 34) aufweist.

5. Dichtung nach einem der Ansprüche 1 bis 4, **dadurch gekennzeichnet, dass** die flexiblen Dome spiegelbildlich zu einem Durchmesser (42) der Wand (14; 94) angeordnet sind.

6. Dichtung nach einem der Ansprüche 1 bis 5, **dadurch gekennzeichnet, dass** zwischen den beiden flexiblen Domen zumindest eine weitere Öffnung (46, 48; 102, 103, 104) in der Wand (14; 94) ausgebildet ist.

7. Dichtung nach Anspruch 6, **dadurch gekennzeichnet, dass** eine erste weitere Öffnung (46) einen Zugang für ein weiteres Instrument, insbesondere eine Optik darstellt.

8. Dichtung nach Anspruch 6 oder 7, **dadurch gekennzeichnet, dass** diese eine zweite weitere Öffnung (48) einen Gasanschluss (56) eingesetzt, aufweist.

9. Dichtung nach einem der Ansprüche 6 bis 8, **dadurch gekennzeichnet, dass** in einer weiteren Öffnung (46) eine Reduzierhülse (58) eingesetzt ist, die proximalseitig eine durchmesserkleinere Öffnung (59) als die weitere Öffnung (46) aufweist.

10. Dichtung nach einem der Ansprüche 1 bis 9, **dadurch gekennzeichnet, dass** in der Kappe (12; 92) eine Klammer (78; 108) integriert ist.

11. Dichtung nach Anspruch 10, **dadurch gekennzeichnet, dass** die Klammer (78; 108) als diametraler Bügel (80) ausgebildet ist, dessen endseitige Krallen (82) am Zugangsinstrument anliegen.

12. Dichtung nach einem der Ansprüche 1 bis 11, **dadurch gekennzeichnet, dass** in einem proximalseitigen Kragen (19, 21) eines flexiblen Domes eine Schlitzdichtung (25, 27) angeordnet ist.

13. Dichtung nach einem der Ansprüche 6 bis 12, **dadurch gekennzeichnet, dass** distalseitig an den weiteren Öffnungen (46, 48; 102, 103, 104) Schlitzdichtungen (49; 105, 106, 107) angeordnet sind.

14. Dichtung nach einem der Ansprüche 10 bis 13, **dadurch gekennzeichnet, dass** die Klammer (78; 108) derart ausgebildet ist, dass Teilkörperabschnitte (62, 64; 122, 124) eines die Zugangsöffnung (70) des Zugangsinstrumentes (60; 120) umgebenden Randes (72; 132) zusammenhaltbar sind.

15. Dichtung nach einem der Ansprüche 1 bis 14, **dadurch gekennzeichnet, dass** von der Kappe (92) zumindest ein Vorsprung vorsteht, der ergreifbar ist und ein Aufstülpen der Kappe (92) auf den Rand (132) der Zugangsöffnung erleichtert.

16. Dichtung nach Anspruch 15, **dadurch gekennzeichnet, dass** mehrere Vorsprünge umfänglich verteilt von der Kappe (92) vorstehen.

17. Dichtung nach Anspruch 15 oder 16, **dadurch gekennzeichnet, dass** der zumindest eine Vorsprung radial nach außen gerichtet von einem unteren Ende (117) der Manschette (96) vorspringt.

18. Dichtung nach einem der Ansprüche 15 bis 17, **dadurch gekennzeichnet, dass** der zumindest eine Vorsprung als Lasche (114, 115, 116, 117) ausgebildet ist.

## Claims

1. Seal for closing-off a proximal-side access port of an access instrument (60; 120) into a body (75), which has a cap (12; 92) having a disc-like wall (14; 94) covering the access port (70) and a circumferential packing (16; 96) which can be put over an edge of the access port, wherein at least two ports are present in the wall, wherein a flexible dome sits on each port which has, on the proximal side, an entry port for the sealed insertion of a shaft of an instrument, **characterized in that** the ports (22, 24) in the wall (14; 94) are designed as approximately circular-segment-shaped ports (22, 24), between which a material web (50) made of the material of the wall (14) is present, and **in that** the proximal side entry port (26, 28) of each dome (14) is smaller than the approximately circular-segment-shaped opening (22, 24) from which each dome (40) raises up.

2. Seal of claim 1, **characterized in that** the flexible dome is designed as a dome-like bellows (18, 20; 98, 100).

3. Seal of claim 2, **characterized in that** the bellows (18, 20; 98, 100) tapers in a Christmas-tree-like fashion (36), as seen across a secant (23) extending along the circular-segment-shaped ports (22, 24).

4. Seal of claims 2 or 3, **characterized in that** all bellows (18, 20; 98, 100) have at least three folds (30, 32, 34).

5. Seal of any one of claims 1 through 4, **characterized in that** the flexible domes are arranged in a mirror-imaged fashion with respect to a diameter (42) of the wall (14; 94).

6. Seal of any one of claims 1 through 5, **characterized in that** at least one further port (46, 48; 102, 103, 104) is formed in the wall (14; 94) between the two flexible domes.

7. Seal of claim 6, **characterized in that** a first further port (46) constitutes an access for a further instrument, in particular an optical system.

8. Seal of claims 6 or 7, **characterized in that** it has a second further port (48), a gas connection (56) being inserted therein.

9. Seal of any one of claims 6 through 8, **characterized in that** a further port (46) has a reducing bush (58) inserted therein, which bush has, on the proximal side, a port (59) with a smaller diameter than the further port (46).

10. Seal of any one of claims 1 through 9, **characterized in that** a clamp (78; 108) is integrated into the cap (12; 92).

11. Seal of claim 10, **characterized in that** the clamp (78; 108) is designed as a diametric strap (80), the claws (82) at the end thereof butting against the access instrument.

12. Seal of any one of claims 1 through 11, **characterized in that** a slit seal (25, 27) is arranged on a proximal-side collar (19, 21) of a flexible dome.

13. Seal of any one of claims 6 through 12, **characterized in that** slit seals (49; 105, 106, 107) are arranged on the distal side of the further ports (46, 48; 102, 103, 104).

14. Seal of any one of claims 10 through 13, **characterized in that** the clamp (78; 108) is designed such that the subsidiary body sections (62, 64; 122, 124) of an edge (72; 132) surrounding the access port (70) of the access instrument (60; 120) can be held together.

15. Seal of any one of claims 1 through 14, **characterized in that** at least one projection projects from the cap (92) which can be grasped and facilitates a drawing of the cap (92) on an edge (132) of the access port.

16. Seal of claim 15, **characterized in that** a plurality of projections projects circumferentially distributed from the cap (92).

17. Seal of claim 15 or 16, **characterized in that** at least one projection projects from a lower end (117) of the packing (96) directed radially outwardly.

18. Seal of any one of claims 15 through 17, **characterised in that** the at least one projection is designed as a flap (114, 115, 116, 117).

## Revendications

1. Joint d'étanchéité, pour sceller une ouverture d'accès du côté proximal d'un instrument d'accès (60; 120) dans un corps (75), comprenant un capuchon (12; 92), qui présente une paroi en forme de disque (14 ; 94) recouvrant l'ouverture d'accès (70) est un manchon périphérique (16 ; 96) qui peut être replié sur le bord de l'ouverture d'accès, au moins deux ouvertures étant prévues dans la paroi, un dôme flexible reposant sur chacune des ouvertures, lequel présente, du côté proximal, une ouverture d'entrée pour introduite de manière hermétique une tige d'un instrument, **caractérisé en ce que** les ouvertures (22, 24) dans la paroi (14 ; 94) sont réalisées sous forme d'ouvertures (22, 24) approximativement en forme de sections de cercle entre lesquelles est prévue une nervure de matériau (50) réalisée dans le matériau de la paroi (14), et **en ce que** l'ouverture d'entrée (26, 28) du côté proximal de chaque dôme (40) est plus petite que l'ouverture (22, 24) approximativement en forme de section de cercle par-dessus laquelle se dressent les dômes respectifs (40).

2. Joint d'étanchéité selon la revendication 1, **caractérisé en ce que** le dôme flexible est réalisé sous forme de soufflet de type dôme (18, 20 ; 98, 100).

3. Joint d'étanchéité selon la revendication 2, **caractérisé en ce que** le soufflet (18, 20 ; 98, 100) se rétrécit en forme de sapin (36) vu transversalement à une sécante (23) s'étendant le long des ouvertures (22, 24) en forme de sections de cercle.

4. Joint d'étanchéité selon la revendication 2 ou 3, **caractérisé en ce que** chaque soufflet (18, 20 ; 98, 100) présente au moins trois plis (30, 32, 34).

5. Joint d'étanchéité selon l'une quelconque des revendications 1 à 4, **caractérisé en ce que** les dômes flexibles sont disposés avec une symétrie spéculaire par rapport à un diamètre (42) de la paroi (14 ; 94).

6. Joint d'étanchéité selon l'une quelconque des revendications 1 à 5, **caractérisé en ce qu'**entre les deux dômes flexibles est réalisée au moins une ouverture supplémentaire (46, 48 ; 102, 103, 104) dans la paroi (14 ; 94).

7. Joint d'étanchéité selon la revendication 6, **caractérisé en ce qu'**une première ouverture supplémentaire (46) constitue un accès pour un autre instrument, notamment un système optique.

8. Joint d'étanchéité selon la revendication 6 ou 7, **caractérisé en ce que** celui-ci présente un raccord de gaz (56) inséré dans une deuxiène ouverture supplémentaire (48).

9. Joint d'étanchéité selon l'une quelconque des revendications 6 à 8, **caractérisé en ce que** dans une ouverture supplémentaire (46) est insérée une douille de réduction (58), qui présente, du côté proximal, une ouverture de plus petit diamètre (59) que l'ouverture supplémentaire (96).

10. Joint d'étanchéité selon l'une quelconque des revendications 1 à 9, **caractérisé en ce que** dans le capuchon (12 ; 92) est intégrée une pince (78 ; 108).

11. Joint d'étanchéité selon la revendication 10, **caractérisé en ce que** la pince (78 ; 108) est réalisée sous forme d'étrier diamétral (80), dont les griffes d'extrémité (82) s'appiquent contre l'instrument d'accès.

12. Joint d'étanchéité selon l'une quelconque des revendications 1 à 11, **caractérisé en ce que** dans un rebord proximal (19, 21) d'un dôme flexible est disposé un joint d'étanchéité à fente (25, 27).

13. Joint d'étanchéité selon l'une quelconque des revendications 6 à 12, **caractérisé en ce que** des joints d'étanchéité à fente (49 ; 105, 106, 107) sont disposés du côté distal au niveau des ouvertures supplémentaires (46, 48 ; 102, 103, 104).

14. Joint d'étanchéité selon l'une quelconque des revendications 10 à 13, **caractérisé en ce que** la pince (78 ; 108) est réalisée de telle sorte que des portions de corps partiel (62, 64 ; 122, 124) d'un bord (72 ; 132) entourant l'ouverture d'accès (70) de l'instrument d'accès (60 ; 120) peuvent être retenues ensemble.

15. Joint d'étanchéité selon l'une quelconque des revendications 1 à 14, **caractérisé en ce qu'**au moins une saillie fait saillie depuis le capuchon (92), laquelle peut être saisie et facilite un enfoncement du capuchon (92) sur le bord (132) de l'ouverture d'accès.

16. Joint d'étanchéité selon la revendication 15, **caractérisé en ce que** plusieurs saillies réparties sur la périphérie font saillie depuis le capuchon (92).

17. Joint d'échanchéité selon la revendication 15 ou 16, **caractérisé en ce que** l'au moins une saillie fait saillie depuis une extrémité inférieure (117) du manchon (96) de manière orientée radialement vers l'extérieur.

18. Joint d'étanchéité selon l'une quelconque des revendications 15 à 17, **caractérisé en ce que** l'au moins une saillie est réalisée sous forme de patte (114, 115, 116, 117).
